(19) Europäisches Patentamt
European Patent Office
Office européen des brevets

(11) Publication number: **0 091 078**
**B1**

(12) # EUROPEAN PATENT SPECIFICATION

(45) Date of publication of patent specification: **26.11.86**

(51) Int. Cl.[4]: **C 07 C 93/197** // A61K31/215

(21) Application number: **83103129.9**

(22) Date of filing: **29.03.83**

(54) **A process for the preparation of derivatives of 2-diethylamino-1-methyl ethyl cis-1-hydroxy-(bicyclohexyl)-2-carboxylate.**

(30) Priority: **01.04.82 IT 2053882**

(43) Date of publication of application:
**12.10.83 Bulletin 83/41**

(45) Publication of the grant of the patent:
**26.11.86 Bulletin 86/48**

(84) Designated Contracting States:
**AT BE CH DE FR GB LI NL SE**

(56) References cited:
**AT-B- 288 348**
**DE-A-2 947 160**

(73) Proprietor: **Laboratori Guidotti S.p.A.**
**Via Trieste 40**
**I-50100 Pisa (IT)**

(72) Inventor: **Sbarbaro, Franco**
**Via Poirè 55 S. Olcese**
**I-16010 Genova (IT)**

(74) Representative: **Bühling, Gerhard, Dipl.-Chem. et al**
**Patentanwaltsbüro Tiedtke-Bühling-Kinne**
**Grupe-Pellmann-Grams-Struif Bavariaring 4**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for the preparation of pharmaceutically acceptable salts of 2-diethylamino-1-methylethyl *cis*-1-hydroxy-(bicyclohexyl)-2-carboxylate and to the salts prepared by said process.

2-Diethylamino-1-methylethyl *cis*-1-hydroxy-(bicyclohexyl)-2-carboxylate, also known under the international common name of Rociverine or "Rociverinum", is an active principle known and used in therapy as an antispastic drug (U.K. Patent No. 1 167 386).

It is prepared by reacting *cis*-1-hydroxy-(bicyclohexyl)-2-carboxylic acid with 1-diethylamino-2-chloropropane in the presence of an aceptor of hydrogen chloride; by this reaction a mixture of position isomers is formed, from which through a thermal treatment, causing an isomerization to take place, and a purification by distillation (see Italian Patent Application No. 30163 A/78), the desired therapeutically active isomer having the formula:

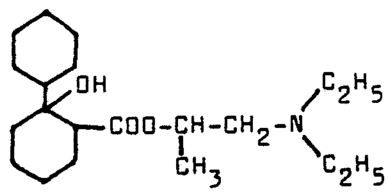

(I)

is obtained. The thus obtained product is a yellow oily liquid not soluble in water having a purity (as determined by gas chromatography) of 98% and is used as such in the pharmaceutical practice for the preparation of vials, tablets and suppositories.

Of course, for pharmaceutical use, an oily substance is of more difficult handling, both in the preparation of the desired formulations and as regards the storage, unless particular cautions are taken.

A further purification of 2-diethylamino-1-methylethyl *cis*-1-hydroxy-(bicyclohexyl)-2-carboxylate according to a known method by fractional distillation under vacuum and at high temperature could only be useful on a laboratory scale and for small amounts, but was fully useless for the production on a commercial scale.

In fact, an extended exposition of Rociverine to high temperature casued its decomposition, 2-diethylamino-1-methylethyl 7-cyclohexyl-7-hydroxyheptanoate being formed. In order to limit such a decomposition, the maximum amount of raw Rociverine which might be purified in this way was not higher than 5 kg, with an obvious great labour expense and thus with a consequent very high cost of the final pure product.

If a fractional distillation under the above conditions was carried out on amounts varying between 30 kg (as the lower limit) and the commercially acceptable quantities, the yield of pure product was reduced to 50% and progressively to lower values.

Attempts were made with more sophisticated equipment (e.g. with the thin-layer distilling apparatus), but without useful results as the compounds to be separated have very close boiling points. However, the distilled product thus obtained, which has a purity (as determined by gas chromatography) of 98%, is a free base and thus not stable under the action of light and air.

The direct salification of Rociverine with an organic acid such as citric acid or maleic acid produces pitch-like substances.

From AT—B—288 348 a process for the preparation of 2-diethylamino-1-methylethyl esters of 2-cyclohexyl-2-hydroxycyclohexanecarboxylic acid is known, in which an alkali salt of 2-cyclohexyl-2-hydroxycyclohexanecarboxylic acid is reacted with a 1,2-dihalogenopropane, the obtained 2-halogeno-1-methylethyl ester is thereafter reacted with 2-diethylamine and, if desired, the obtained 2-diethylamino-1-methylethyl ester is converted into an addition salt by an acid, e.g. succinic acid or citric acid. Accordingly the ester is directly converted by salification into an addition salt without preparing an intermediate product. The salification products thus obtained are reported to be generally in the form of oils or uncrystallizable amorphous products and only in some cases in the form of crystalline products.

It is the object of the present invention to provide a process for the preparation of pharmaceutically acceptable salts of 2-diethylamino-1-methylethyl *cis*-1-hydroxy-(bicyclohexyl)-2-carboxylate in form of solids having a purity of more than 99% with physical properties suitable for the therapeutical use, i.e. of solids which can be easily handled when preparing pharmaceutical compositions based thereon, the process eliminating complex and expensive purification steps.

The object of the present invention is achieved by a process in which the phosphate of 2-diethylamino-1-methylethyl *cis*-1-hydroxy-(bicyclohexyl)-2-carboxylate is prepared, and then the phosphate is converted into the desired salt.

In the process of the present invention, the starting product is not the distilled Rociverine but the raw product of the reaction for the preparation of the active compound.

The desired pharmaceutically acceptable salts are obtained by a process wherein 2-diethylamino-1-methylethyl *cis*-1-hydroxy-(bicyclohexyl)-2-carboxylate is reacted with phosphoric acid in an organic solvent and at low temperature, the base from the alkalinized aqueous solution is extracted with an organic solvent, and thereafter the salification is carried out with a pharmaceutically acceptable organic acid in an organic solvent and at low temperature or by treatment of the aqueous solution of the phosphate salt of Rociverine with an aqueous solution of disodium pamoate.

The process is based on the salification with phosphoric acid, dissolved in a suitable solvent, for instance acetone, of the Rociverine base, either raw or distilled, the latter being also dissolved in a suitable solvent. By cooling a white crystalline solid is obtained, highly soluble in water, stable and not hygroscopic, having a melting point of 89—92°C and a parity (as determined by gas chromatography) of more than 99%.

The pharmaceutically useful salts of Rociverine, with a purity of more than 99%, are obtained by starting from Rociverine phosphate, extracting with a suitable solvent, for instance ethyl ether, the base after alkalinization of the aqueous solution of the phosphate, salifying with the desired acid, in anhydrous form, dissolved in a suitable solvent, for instance acetone, and cooling; the salt with pamoic acid is obtained by mixing the aqueous solution of Rociverine phosphate with the aqueous solution of disodium pamoate.

In the case of Rociverine citrate, a white powder, highly soluble in water and very little hygroscopic, is obtained, having a melting point of 82—84°C and a purity (as determined by gas chromatography) of more than 99%.

In the case of Rociverine maleate, there is obtained a white crystalline powder, highly soluble in water and very little hygroscopic, having a melting point of 79—81°C.

In the case of Rociverine fumarate, there is obtained a white crystalline powder, soluble in water and non hygroscopic, having a melting point of 111—113°C.

In the case of Rociverine pamoate, there is obtained a white powder, not soluble in water, non hygroscopic and having a melting point of 103—105°C.

The following examples, having illustrative purpose, detailedly describe the process of the invention with reference to the citrate, it being understood that for the other pharmaceutically acceptable salts (apart from pamoate) the process is carried out likewise.

Example 1

In a three-neck flask, having a stirrer and a dropping funnel, 339 g of 2-(diethylamino)-1-methylethyl *cis*-1-hydroxy-(bicyclohexyl)-2-carboxylate (Rociverine) are dissolved in 250 mls of acetone and the flask is placed in a refrigerating bath at a temperature of between 0°C and −20°C.

There are dropwise added under stirring over about 30 minutes 115 g of 85% phosphoric acid dissolved in 300 mls of acetone.

Upon completion of the addition, the mixture is stirred for 2 hours.

The precipitated product is filtered, washed with acetone and dried in an oven under vacuum. Rociverine phosphate is obtained as microcrystalline solid having a melting point of 89—92°C with a purity (as determined by gas chromatography) of 99% and a yield of 93%.

Example 2

432 g of Rociverine phosphate are dissolved in 2 parts of water, and the mixture is alkalinized with aqueous solution of sodium hydroxide and extracted with a suitable solvent, for instance ethyl ether; the ether is then dried over anhydrous sodium sulphate, cooled to 0 to 5°C, and added under stirring with 192 g of citric acid dissolve in the minimum amount of acetone.

The mixture is maintained under stirring for a minimum of 2 hours, so as to permit the salt to completely precipitate, filtered, washed with the same solvent and dried under vacuum.

There is thus obtained in a yield of more than 95% of Rociverine citrate in form of a white powder, very little hygroscopic, having a melting point of 82—84°C and a purity (as determined by gas chromatography) of more than 99%.

Example 3

Starting again from 432 g of Rociverine, the process of example 2 is repeated by adding under stirring to the ethereal solution of the base, cooled at 0°C to 5°C, 116 g of fumaric acid (or 116 g of maleic acid) dissolved in the minimum amount of acetone.

The isolation is then carried out as described in example 2, resulting in this case to a yield of more than 95% of Rociverine fumarate as a white, non hygroscopic powder, having a melting point of 111—113°C and a purity (as determined by gas chromatography) of more than 99%; in the case of maleic acid there is obtained a white crystalline powder, very little hygroscopic, having a melting point of 79 to 81°C.

Example 4

A solution of 432 g of Rociverine phosphate in 20 parts of water is slowly added under stirring into a solution obtained by dissolving 194,2 g of pamoic acid in 3 litres of water containing 40 g of NaOH. The thus formed precipitate is collected and washed by resuspending it in water.

The mixture is filtered giving place, with a yield of 95%, to a yellowish powder comprising a compound

formed by two molecules of Rociverine and one molecule of pamoic acid, with a melting point of 103—105°C. It is important to point out that the Rociverine phosphate has no substantial therapeutical properties, so that the preparation thereof would not have had any therapeutical purpose. Likewise it might not be supposed that by preparing the phosphate salt and then converting it into another pharmaceutically acceptable salt, as the citrate, the purposes of the present invention would be achieved, whereas the direct preparation of Rociverine citrate was giving place to a product having different properties and physical characteristics.

Furthermore the process of the invention, namely the step of conversion to the phosphate, permits novel compounds to be prepared, which are therapeutically interesting, namely fumarate, maleate and pamoate, which are not directly obtainable from the base, even if distilled.

**Claims**

1. A process for the preparation of pharmaceutically acceptable salts of 2-diethylamino-1-methylethyl *cis*-1-hydroxy-(bicyclohexyl)-2-carboxylate, characterized in that the phosphate of 2-diethylamino-1-methylethyl *cis*-1-hydroxy-(bicyclohexyl)-2-carboxylate is prepared, and then the phosphate is converted into the desired salt.

2. A process according to claim 1, characterized in that 2-diethylamino-1-methylethyl *cis*-1-hydroxy-(bicyclohexyl)-2-carboxylate is reacted with phosphoric acid in an organic solvent and at low temperature, the resulting precipitate is made alkaline and extracted with an organic solvent, and thereafter the salification is carried out with an anhydrous organic acid in an organic solvent and at low temperature, said acid being selected from citric, maleic and fumaric acids.

3. A process according to claim 2, characterized in that the reaction with phosphoric acid is carried out in acetone and the reaction temperature is between 0°C and −20°C.

4. A process according to claim 2, characterized in that said alkalinization is carried out with an alkali hydroxide and said extraction is carried out with ethyl ether.

5. A process according to claim 2, characterized in that the salification reaction is caried out with citric acid in acetone and at a temperature of between 0°C and −5°C.

6. A process according to claim 1, characterized in that 2-diethylamino-1-methylethyl *cis*-1-hydroxy-(bicyclohexyl)-2-carboxylate is reacted with phosphoric acid in an organic solvent and at low temperature and the resulting precipitate is reacted with a salt of pamoic acid with a strong base in alkaline medium.

7. Phosphate of 2-diethylamino-1-methylethyl *cis*-1-hydroxy-(bicyclohexyl)-2-carboxylate.

8. Fumarate of 2-diethylamino-1-methylethyl *cis*-1-hydroxy-(bicyclohexyl)-2-carboxylate in crystalline form.

9. Maleate of 2-diethylamino-1-methylethyl *cis*-1-hydroxy-(bicyclohexyl)-2-carboxylate in crystalline form.

10. Pamoate of 2-diethylamino-1-methylethyl *cis*-1-hydroxy-(bicyclohexyl)-2-carboxylate.

**Patentansprüche**

1. Verfahren zur Herstellung pharmazeutisch verträglicher Salze von 2-Diethylamino-1-methylethyl-*cis*-1-hydroxy-(bicyclohexyl)-2-carboxylat, dadurch gekennzeichnet, daß das Phosphat von 2-Diethylamino-1-methylethyl-*cis*-1-hydroxy-(bicyclohexyl)-2-carboxylat hargestellt und das Phosphat dann in das gewünschte Salz umgewandelt wird.

2. Verfahren nach Anspruch 1, dadurch gekennzeichnet, daß-2-Diethylamino-1-methylethyl-*cis*-1-hydroxy-(bicyclohexyl)-2-carboxylat in einem organischen Lösungsmittel und bei niedriger Temperatur mit Phosphorsäure zur Reaktion gebracht wird, der erhaltene Niederschlag alkalisch gemacht und mit einem organischen Lösungsmittel extrahiert wird und danach die Salzbildung mit einer wasserfreien organischen Säure in einem organischen Lösungsmittel und bei niedriger Temperatur durchgeführt wird, wobei die erwähnte Säure aus Citronensäure, Maleinsäure und Fumarsäure ausgewählt wird.

3. Verfahren nach Anspruch 2, dadurch gekennzeichnet, daß die Reaktion mit Phosphorsäure in Aceton durchgeführt wird und daß die Reaktionstemperatur zwischen 0°C und −20°C liegt.

4. Verfahren nach Anspruch 2, dadurch gekennzeichnet, daß die erwähnte Alkalisierung mit einem Alkalimetallhydroxid durchgeführt wird und daß die erwähnte Extraktion mit Ethylether durchgeführt wird.

5. Verfahren nach Anspruch 2, dadurch gekennzeichnet, daß die Salzbildungsreaktion in Aceton und bei einer Temperatur zwischen 0°C und −5°C mit Citronensäure durchgeführt wird.

6. Verfahren nach Anspruch 1, dadurch gekennzeichnet, daß 2-Diethylamino-1-methylethyl-*cis*-1-hydroxy-(bicyclohexyl)-2-carboxylat in einem organischen Lösungsmittel und bei niedriger Temperatur mit Phosphorsäure zur Reaktion gebracht wird und der erhaltene Niederschlag in alkalischem Medium mit einem Salz der Embonsäure mit einer starken Base zur Reaktion gebracht wird.

7. Phosphat von 2-Diethylamino-1-methylethyl-*cis*-1-hydroxy-(bicyclohexyl)-2-carboxylat.

8. Fumarat von 2-Diethylamino-1-methylethyl-*cis*-1-hydroxy-(bicyclohexyl)-2-carboxylat in kristalliner Form.

9. Maleat von 2-Diethylamino-1-methylethyl-*cis*-1-hydroxy-(bicyclohexyl)-2-carboxylat in kristalliner Form.

10. Embonat von 2-Diethylamino-1-methylethyl-*cis*-1-hydroxy-(bicyclohexyl)-2-carboxylat.

**Revendications**

1. Procédé de préparation de sels pharmaceutiquement acceptables de 2-diéthylamino-1-méthyléthyl *cis*-1-hydroxy-(bicyclohexyl)-2-carboxylate, caractérisé en ce que l'on prépare le phosphate de 2-diéthylamino-1-méthyléthyl *cis*-1-hydroxy-(bicyclohexyl)-2-carboxylate, et l'on transforme ensuite le phosphate en le sel désiré.

2. Procédé selon la revendication 1, caractérisé en ce qu'on fait réagir le 2-diméthylamino-1-méthyléthyl *cis*-1-hydroxy-(bicyclohexyl)-2-carboxylate avec de l'acide phosphorique dans un solvant organique et à basse température, on alcalinise le précipité résultant et on l'extrait au moyen d'un solvant organique et l'on effectue ensuite la salification avec un acide organique anhydre dans un solvant organique et à basse température, en choisissant ledit acide parmi l'acide citrique, l'acide maléique et l'acide fumarique.

3. Procédé selon la revendication 2, caractérisé en ce qu'on effectue la réaction avec l'acide phosphorique dans de l'acétone, et en ce que la température de réaction est comprise entre 0° et −20°C.

4. Procédé selon la revendication 2, caractérisé en ce qu'on effectue ladite alcalinisation avec un hydroxyde alcalin, et l'on effectue ladite extraction avec de l'éther éthylique.

5. Procédé selon la revendication 2, caractérisé en ce qu'on effectue la réaction de salification avec de l'acide citrique dans de l'acétone et à une température comprise entre 0°C et −5°C.

6. Procédé selon la revendication 1, caractérisé en ce qu'on fait réagir le 2-diéthylamino-1-méthyléthyl *cis*-1-hydroxy-(bicyclohexyl)-2-carboxylate avec de l'acide phosphorique dans un solvant organique et à basse température, et l'on fait réagir le précipité resultant sur un sel d'acide pamoïque avec une base forte en milieu alcalin.

7. Phosphate de 2-diéthylamino-1-méthyléthyl *cis*-1-hydroxy-(bicyclohexyl)-2-carboxylate.

8. Fumarate de 2-diéthylamino-1-méthyléthyl *cis*-1-hydroxy-(bicyclohexyl)-2-carboxylate sous forme cristalline.

9. Maléate de 2-diéthylamino-1-méthyléthyl *cis*-1-hydroxy-(bicyclohexyl)-2-carboxylate sous forme cristalline.

10. Pamoate de 2-diéthylamino-1-méthyléthyl *cis*-1-hydroxy-(bicyclohexyl)-2-carboxylate.